# EUROPÄISCHE PATENTSCHRIFT

(11) **EP 2 801 825 B1**
(45) Veröffentlichungstag und Bekanntmachung des Hinweises auf die Patenterteilung: **18.11.2015**
(21) Anmeldenummer: 13002450.8
(22) Anmeldetag: 08.05.2013
(51) Int. Cl.: C12Q 1/04

(54) **Massenspektrometrische Bestimmung der Resistenzen von Mikroben**
Mass spectrometric determination of the resistances of microbes
Détermination par spectrométrie de masse des résistances de microbes

(43) Veröffentlichungstag der Anmeldung: 12.11.2014
(73) Patentinhaber: Bruker Daltonik GmbH, 28359 Bremen (DE)
(72) Erfinder: Sparbier, Katrin, 28357 Bremen (DE); Lange, Christoph, 28879 Grasberg (DE); Franzen, Jochen, 28357 Bremen (DE); Kostrzewa, Markus, 28865 Lilienthal (DE)

(56) Entgegenhaltungen:
- DE-B4-102006 021 493
- US-A1- 2011 300 552
- ONG S-E ET AL: "A practical recipe for stable isotope labeling by amino acids in cell culture (SILAC)", NATURE PROTOCOLS, NATURE PUBLISHING GROUP, GB, Bd. 1, Nr. 6, 1. Januar 2007 (2007-01-01), Seiten 2650-2660, XP002570015, ISSN: 1750-2799, DOI: 10.1038/NPROT.2006.427 [gefunden am 2007-01-11]
- HARSHA H C ET AL: "Quantitative proteomics using stable isotope labeling with amino acids in cell culture", NATURE PROTOCOLS, NATURE PUBLISHING GROUP, GB, Bd. 3, 6. März 2008 (2008-03-06), Seiten 505-516, XP009148116, ISSN: 1750-2799, DOI: 10.1038/NPROT.2008.2 [gefunden am 2008-03-06]
- PLAMEN A. DEMIREV ET AL: "Establishing Drug Resistance in Microorganisms by Mass Spectrometry", JOURNAL OF THE AMERICAN SOCIETY FOR MASS SPECTROMETRY, Bd. 24, Nr. 8, 9. April 2013 (2013-04-09), Seiten 1194-1201, XP055077749, ISSN: 1044-0305, DOI: 10.1007/s13361-013-0609-x
- MANN M: "Functional and quantitative proteomics using SILAC", NATURE REVIEWS MOLECULAR CELL BIOLOGY 20061208 GB, Bd. 7, Nr. 12, 8. Dezember 2006 (2006-12-08), Seiten 952-958, XP009172514, ISSN: 1471-0072
- DILWORTH D J ET AL: "QTIPS: A Novel Method of Unsupervised Determination of Isotopic Amino Acid Distribution in SILAC Experiments", JOURNAL OF THE AMERICAN SOCIETY FOR MASS SPECTROMETRY, ELSEVIER SCIENCE INC, US, Bd. 21, Nr. 8, 1. August 2010 (2010-08-01) , Seiten 1417-1422, XP027182164, ISSN: 1044-0305 [gefunden am 2010-04-08]

## Beschreibung

Die Erfindung betrifft ein massenspektrometrisches Verfahren zur Bestimmung der mikrobiellen Resistenzen gegen Antibiotika unter Verwendung isotopenmarkierter Nahrungsbausteine.

### Stand der Technik

In dieser Schrift wird statt der gesetzlichen "vereinheitlichten atomaren Masseneinheit" (u) die Einheit "Dalton" (Da) verwendet, die in der letzten (achten) Ausgabe 2006 der Schrift "The International System of Units (SI)" des "Bureau International des Poids et Mesures" der atomaren Masseneinheit gleichwertig beigestellt wurde; vor allem, wie dort angemerkt, um die Einheiten Kilodalton, Millidalton und Ähnliche verwenden zu können.

Aus Gründen der Vereinfachung wird in dieser Schrift nur der Begriff "Proteine" verwendet, obwohl im hier betrachteten Massenbereich die Proteine oft besser als "Peptide" zu bezeichnen wären. Der Übergang der leichteren Peptide zu den schwereren Proteinen ist aber fließend und nicht eindeutig definiert.

Der Begriff "Antibiotikum" umfasst hier pharmakologische Wirkstoffe für die Behandlung bakterieller Infektionskrankheiten und andere antibakterielle Substanzen, etwa zur Desinfektion.

Die Erfolge des Penicillins, aber auch das Auftreten der ersten Resistenzen führten zur Suche und Entdeckung vieler weiterer Antibiotika. Seit Penicillin als erster pharmakologischer Wirkstoff eingesetzt wurde, haben Bakterienstämme in zunehmendem Maße verschiedenartige Resistenzen entwickelt, sich also Eigenschaften angeeignet, die es ihnen ermöglichen, die Wirkung von antibiotisch wirkenden Substanzen abzuschwächen oder ganz zu neutralisieren. Resistenzen sind inzwischen häufig: in den USA sind etwa 70% der in Krankenhäusern erworbenen infektiösen Keime resistent gegen mindestens ein Antibiotikum. Oft sind Patienten mit Bakterienstämmen infiziert, die gegen mehrere Antibiotika resistent sind (Multiresistenz). Sogenannte Problemkeime sind dabei vor allem der Methicillin-resistente Staphylococcus aureus (MRSA), Pseudomonas spec., Escherichia coli mit ESBL-Resistenz und Mycobacterium tuberculosis. Schätzungen des CDC (Center for Disease Control and Prevention) gehen für die USA von zwei Millionen in Krankenhäusern erworbenen Infektionen für das Jahr 2004 aus, mit etwa 90 000 Todesfällen.

Die Gründe für die Zunahme der Resistenzen sind vielfältig: leichtfertige Verschreibungen von Antibiotika, selbst wenn das nicht nötig wäre; leichtfertig abgebrochene Therapien; leichtfertige, oft rein vorbeugende Anwendungen in der Land- und Viehwirtschaft. Alle diese Verhaltensweisen helfen der Auslese und Verbreitung von resistenten Bakterienstämmen gegenüber nicht resistenten Bakterienstämmen.

Der Erfolg einer Therapie bei bakteriellen Infektionen, die in Akutsituationen wie einer Sepsis, oder als Sekundärinfektion bei einer schon vorhandenen Primärerkrankung (oder Primärinfektion) lebensbedrohlich sein können, hängt oft davon ab, dass schon die erste Verabreichung eines Antibiotikums wirksam ist. Eine gezielte Verabreichung setzt nicht nur eine möglichst schnelle und fehlerfreie Identifizierung des Krankheitserregers voraus, sondern auch eine schnelle Bestimmung von dessen Resistenz gegen verschiedene Antibiotika.

In der mikrobiologischen Routine wird heutzutage die Resistenz von Mikroben dadurch bestimmt, dass Mikroben aus einer zu untersuchenden Probe in vitro auf Nährmedien (z.B. Agarplatten) oder in Nährmedien (z.B. Bouillonkulturen) kultiviert werden, denen jeweils ein Antibiotikum zugegeben wird. Ob die Mikroben sich unter dem Einfluss des Antibiotikums vermehren, also resistent gegenüber dem Antibiotikum sind, wird visuell mit dem Auge oder automatisiert mit optischen Mitteln bestimmt. Das auf einer optischen Auswertung beruhende Routineverfahren ermöglicht eine einfache, aber zeitaufwendige Bestimmung der Resistenz, da die Kultivierung bis zu einer optisch wahrnehmbaren Wirkung durchgeführt werden muss. Dieses Verfahren dauert in der Regel 24 bis 48 Stunden. Ein Vorteil dieses Routineverfahrens besteht darin, dass die Wirksamkeit bzw. die Unwirksamkeit eines Antibiotikums gegenüber den Mikroben einer Probe direkt gemessen wird (es handelt sich um einen "funktionellen Test").

Es werden in der Regel Kultivierungsansätze mit abgestuften Konzentrationen des Antibiotikums getestet, um die "minimale Hemm-Konzentration" (MHK) zu bestimmen. Die minimale Hemm-Konzentration bezeichnet die niedrigste Konzentration einer Substanz, bei der die Vermehrung eines Mikroorganismus visuell nicht mehr wahrgenommen werden kann. In der Regel wird die MHK bestimmt, einige Antibiotika können aber auch über die "minimale bakterizide Konzentration" (MBK) charakterisiert werden, bei der innerhalb eines festen Zeitraums 99,9 % der Erreger abgetötet werden. Während die MHK prinzipiell bei jedem Antibiotikum ermittelt werden kann, ist die MBK nur bei jenen sinnvoll, die nicht nur eine hemmende, sondern auch eine abtötende (bakterizide) Wirkung entfalten können. Dies sind beispielsweise Aminoglykoside, Gyrasehemmer, Penicilline. Nach der Resistenzbestimmung werden die nachgewiesenen Keime als S - sensibel, I - Intermediär oder R - resistent bezeichnet.

Neben der Kultivierung bei Anwesenheit von Antibiotika gibt es auch genetische Verfahren zur Bestimmung von Resistenzen. Der Nachweis einer Resistenz erfolgt dabei durch den Nachweis von bekannten Resistenzgenen im Genom des jeweiligen Krankheitserregers. Ein Vorteil der genetischen Verfahren besteht darin, dass die Resistenzgene durch Techniken wie der Polymerasen-Kettenreaktion (PCR) vervielfältigt werden können und damit die Analysedauer nicht mehr durch die Vermehrungsrate der Bakterien bestimmt wird. Die Nachteile bestehen darin, dass sie im Vergleich zum Routineverfahren mit höheren Kosten verbunden sind und keine funktionellen Tests darstellen. So kann zwar ein Resistenzgen vorhanden sein, aber nicht exprimiert werden, wodurch der zu untersuchende Bakterienstamm nicht resistent ist, aber durch das Verfahren als resistent erkannt wird.

Viele Arten von Mikroorganismen, insbesondere Bakterien und einzellige Pilze wie Hefen, lassen sich heutzutage schnell und mit geringen Fehlerraten massenspektrometrisch identifizieren. Unter dem Begriff "Identifizierung" ist dabei die taxonomische Klassifizierung, also die Bestimmung von Familie, Gattung und Art zu verstehen. Die Identifizierung erfolgt im Routinebetrieb durch eine Ähnlichkeitsanalyse zwischen einem MALDI-Massenspektrum (MALDI = Matrix Assisted Laser Desorption/Ionization) der zu untersuchenden Probe und MALDI-Referenzspektren von bekannten Mikroorganismen. Jedem der Referenzspektren wird in der Ähnlichkeitsanalyse eine Kennzahl zugeordnet, die ein Maß für die Übereinstimmung des entsprechenden Referenzspektrums mit dem Massenspektrum der Probe ist. Überschreiten die Ähnlichkeitswerte bestimmte Grenzwerte, so können Familien, Gattung, Spezies oder sogar Stamm identifiziert werden. Dieses Identifizierungsverfahren für Mikroorganismen hat sich sowohl in groß angelegten Studien wie auch im täglichen Einsatz in vielen mikrobiologischen Laboratorien als außerordentlich erfolgreich erwiesen. Je nach Apparatur können 48 bis 384 Mikrobenproben zeitparallel bestimmt werden; ab Aufzucht einer Kolonie dauert es nur Minuten bis zur Identifizierung. Das Verfahren ist daher schnell und kostengünstig. Es hat sehr geringe Fehlerraten, weit geringer als die herkömmlicher mikrobiologischer Identifizierungsverfahren. Neuere Studien belegen, dass dieses massenspektrometrische Identifizierungsverfahren zuverlässiger richtige Ergebnisse liefert als die DNA-Analyse, die bisher als der "Goldstandard" angesehen wurde. Es gibt inzwischen Massenspektrometer, zugehörige Auswerteprogramme und Bibliotheken mit Referenzspektren auf dem Markt, die nach Medizinproduktegesetz und anderen, nationalen oder internationalen Vorschriften und Richtlinien als IVD-Produkte für medizinische Diagnostik zugelassen sind.

Es gibt Ansätze, die massenspektrometrische Identifizierung von Mikroorganismen auf eine massenspektrometrische Bestimmung ihrer Resistenzen zu erweitern. Leider hat sich herausgestellt, dass es bisher nur in seltenen Ausnahmefällen möglich war, die Resistenzen aus einem Massenspektrum direkt zu bestimmen, obwohl sich die Resistenzen auch im Vorhandensein neuer oder veränderter Proteine ausdrücken müssten.

Aus der Patentschrift DE 10 2006 021 493 B4 (V. M. Govorun und J. Franzen, 2006, entsprechend GB 2438066 B, US 8,293,496 B2; im Folgenden als "Govorun" bezeichnet) sind massenspektrometrische Verfahren zur Resistenzbestimmung von Bakterien bekannt, in denen Proteinprofile der Bakterien nach Kultivierungen mit und ohne Zugabe von Antibiotika massenspektrometrisch gemessen und verglichen werden. Wie die Schrift ausführt, kann die Resistenz unter anderem dadurch erkannt werden, dass die Mikroben auch in Anwesenheit von Antibiotika weiter leben und dabei Nahrung aus dem Medium aufnehmen. Sind dabei isotopenmarkierte Nahrungsbestandteile im Medium enthalten, so zeigen die dadurch veränderten Massenspektren die Resistenz an. Nicht-resistente Mikroben dagegen erleiden eine Wachstumshemmung oder eine strukturelle Zerstörung und nehmen keine solchen Nahrungsbestandteile mehr auf. Das Verfahren von Govorun ist wie das oben beschriebene Routineverfahren ein funktioneller Test, führt aber schneller zu einer messbaren Aussage als die optischen Standardverfahren.

In der Arbeit "Establishing Drug Resistance in Microorganisms by Mass Spectrometry", (P. A. Demirev et al.; J. Am. Soc. Mass Spectrom. (2013)) wird eine Ausführungsform des Verfahrens von Govorun beschrieben, in der Mikroben unter Zugabe von Antibiotika in einem Medium kultiviert werden, in denen alle ¹²C-Atome durch ¹³C ersetzt sind. Die möglichen Verschiebungen der massenspektrometrischen Peaks (Massensignale) in den Massenspektren der Mikroben aus diesen Kulturen gegenüber üblichen Referenzspektren werden durch vier verschiedene Verfahren zumindest näherungsweise vorausberechnet, darunter als einfachstes Verfahren eine näherungsweise Vorausberechnung durch den mittleren Gehalt an C-Atomen für Proteine gegebener Masse; kompliziertere Verfahren suchen eine de-novo-Sequenzierung durch Tandem-MS oder eine Identifizierung über Protein-Datenbanken. Nur bei Resistenz sollten diese verschobenen Peaks zu finden sein, weil nur resistente Mikroben bei Anwesenheit von Antibiotika wachsen können. Dieses Verfahren ist jedoch wegen der Verwendung eines durch und durch isotopenmarkierten Mediums ausgesprochen teuer; außerdem ist die Voraussage der Peakverschiebungen entweder ungenau oder aufwändig.

In der Offenlegungssschrift WO 2011/152899 Al (P. A. Demirev et al.) werden, wie die Zusammenfassung angibt, Massenspektren von Mikroben oder von isolierten Biomarkern aus Mikroben, die in einem isotopenmarkierten Medium mit einem Antibiotikum gewachsen sind, mit Massenspektren von Mikroben oder Biomarkern aus Mikroben verglichen, die in Normalmedien ohne Antibiotikum gewachsen sind. Die Resistenz wird bestimmt durch Vorhersage und Detektion einer charakteristischen Massenverschiebung, die anzeigt, dass die Mikrobe in der Gegenwart eines Antibiotikums wächst und dabei isotopenmarkiertes Material in einen oder mehrere Biomarker aufnimmt, wodurch die Massenverschiebung eintritt.

### Aufgabe der Erfindung

Es ist die Aufgabe der Erfindung, ein massenspektrometrisches Verfahren bereitzustellen, mit dem eine Resistenz von Mikroben gegenüber einem oder auch mehreren Antibiotika relativ schnell (möglichst in weniger als acht Stunden), sicher und vor allem auch preiswert bestimmt werden kann. Das Verfahren soll möglichst in dem Routine-Massenspektrometer durchgeführt werden können, das auch für Identifizierungen benutzt wird.

### Beschreibung der Erfindung

Die Erfindung stellt ein erstes erfindungsgemäßes Verfahren zur massenspektrometrischen Bestimmung der Resistenz von Mikroben bereit, in dem die Mikroben aus einer zu untersuchenden Probe in einer ersten und einer zweiten Kultur, in denen ein Medium mit isotopenmarkierten Nahrungsbausteine verwendet wird, mit bzw. ohne Zugabe eines Antibiotikums kultiviert werden und danach Massenspektren der Mikroben aus den beiden Kulturen aufgenommen und miteinander verglichen werden, wobei das Medium ein synthetisches Medium ist, das proteinogene Aminosäuren enthält, und mindestens eine der Aminosäuren isotopenmarkiert ist und alle isotopenmarkierten Aminosäuren die gleiche ganzzahlige Massendifferenz *Δm* zu den entsprechenden unmarkierten Aminosäuren aufweisen. Der Begriff "Aminosäure" wird im Folgenden gleichbedeutend mit Aminosäurensorte verwendet.

Das im Verfahren verwendete synthetische Medium enthält bevorzugt nur eine isotopenmarkierte Aminosäure, die in ribosomalen Proteinen der Mikroben weniger als fünf Prozent, insbesondere weniger als ein Prozent ausmacht. Das synthetische Medium kann auch mehrere isotopenmarkierte Aminosäuren enthalten, wobei diese isotopenmarkierten Aminosäuren in ribosomalen Proteinen der Mikroben insgesamt weniger als zehn Prozent, insbesondere weniger als fünf Prozent ausmachen. Das synthetische Medium enthält neben den Aminosäuren weiterhin bevorzugt Kohlenhydrate, Vitaminen und Mineralien.

Die Mikroben der zu untersuchenden Probe können zusätzlich in einer dritten Kultur im synthetischen Medium kultiviert werden, das nur unmarkierte Aminosäuren und kein Antibiotikums enthält. Es wird ein Massenspektrum der Mikroben aus der dritten Kultur aufgenommen und mit dem Massenspektrum der ersten Kultur verglichen, wobei die Mikroben als resistent ausgewiesen werden, wenn das Massenspektrum der ersten Kultur (mit Antibiotikum/mit mindestens einer isotopenmarkierten Aminosäure) dem Massenspektrum der zweiten Kultur (ohne Antibiotikum/ mit mindestens einer isotopenmarkierten Aminosäure) ähnlicher als dem Massenspektrum der dritten Kultur ist. Die Ähnlichkeit zwischen den Massenspektren kann durch unterschiedliche mathematisch-statistische Analyseverfahren ermittelt werden, wie beispielsweise durch eine Kreuzkorrelation, ANN (Artificial Neural Network Analysis), PCA (Principal Component Analysis, Hauptkomponentenanalyse), PLS-DA (Partial Least-Square Discriminant Analysis), SVM (Support Vektor Machines), hierarchische Cluster-Analysen oder andere unüberwachte oder überwachte Klassifizierungstechniken. Sollten für ein mathematisch-statistisches Analyseverfahren mehr als die drei Massenspektren nötig sein, können von Mikroben der drei Kulturen jeweils mehrere Wiederholungsspektren aufgenommen werden.

Für die Prüfung der Resistenz gegenüber mehreren Antibiotika müssen nach dem ersten Verfahren mehrere isotopenmarkierte Kulturen mit mehreren Antibiotika angesetzt werden, erforderlichenfalls sogar mit jeweils verschiedenen Konzentrationsabstufungen der Antibiotika. Da der Verbrauch einer solch großen Zahl von Kulturen mit einem isotopenmarkierten Medium (mit jeweils mindestens einer isotopenmarkierten Aminosäure) teuer ist, wird hier ein zweites erfindungsgemäßes Verfahren vorgeschlagen, das etwas längere Zeit braucht, aber kostengünstiger ist.

Das zweite erfindungsgemäße Verfahren zur massenspektrometrischen Bestimmung der Resistenz von Mikroben umfasst folgende Verfahrensschritte: (a) Kultivierung der Mikroben in einem synthetischen Medium, in dem mindestens eine der Aminosäuren isotopenmarkiert ist und alle isotopenmarkierten Aminosäuren in dem Medium die gleiche ganzzahlige Massendifferenz *Δm* zu den entsprechenden unmarkierten Aminosäuren aufweisen, (b) Kultivierung der in Schritt (a) kultivierten Mikroben in einer ersten und einer zweiten Kultur mit einem Normalmedium ohne isotopenmarkierte Nahrungsbausteine, jeweils mit bzw. ohne Zugabe eines Antibiotikums, und (c) Bestimmung der Resistenz gegenüber dem Antibiotikum durch Aufnahme und Vergleich von Massenspektren der Mikroben der ersten und zweiten Kultur.

Das zweite Verfahren besteht im Wesentlichen darin, zunächst in einem ersten Schritt in einer ersten Kultur in einem Medium mit mindestens einer isotopenmarkierten Aminosäure, Kohlenhydraten, Vitaminen und Mineralien genügend Mikroben zu generieren, bei denen die betreffenden Aminosäuren praktisch vollständig durch die isotopenmarkierten Aminosäuren ersetzt sind. Im zweiten Schritt werden diese isotopenmarkierten Mikroben dann in nicht isotopenmarkierten Normalmedien kultiviert, die preiswert sind und zugleich optimale Wachstumsbedingungen bieten können. Die isotopenmarkierten Mikroben können nun zeitgleich in einem preiswerten Normalmedium ohne Antibiotika und in mehreren preiswerten Normalmedien mit verschiedenen Antibiotika, erforderlichenfalls jeweils in mehreren Konzentrationsabstufungen, kultiviert werden. Resistente Mikroben bauen jetzt neue Proteine mit unmarkierten Aminosäuren auf, wobei die Proteine mit isotopenmarkierten Aminosäuren immer stärker verdünnt werden; nicht-resistente Mikroben mit Wachstumshemmung behalten den Großteil ihrer Proteine mit isotopenmarkierten Aminosäuren.

Auch im zweiten Verfahren ist es günstig, nur eine oder wenige seltenere oder durchschnittlich häufige Aminosäuren in isotopenmarkierter Form zu verwenden. Das im ersten Kultivierungsschritt (a) verwendete synthetische Medium enthält bevorzugt nur eine isotopenmarkierte Aminosäure, die in ribosomalen Proteinen der Mikroben weniger als fünf Prozent, insbesondere weniger als ein Prozent ausmacht. Das synthetische Medium kann auch mehrere isotopenmarkierte Aminosäuren enthalten, wobei diese isotopenmarkierten Aminosäuren in ribosomalen Proteinen der Mikroben insgesamt weniger als zehn Prozent, insbesondere weniger als fünf Prozent ausmachen.

Ähnlich wie beim ersten Verfahren kann ein drittes Massenspektrum von den in Schritt a) kultivierten Mikroben aufgenommen und mit dem Massenspektrum der ersten Kultur verglichen werden, wobei die Mikroben als resistent ausgewiesen werden, wenn das Massenspektrum der ersten Kultur (mit Antibiotikum) dem Massenspektrum der zweiten Kultur (ohne Antibiotikum) ähnlicher als dem dritten Massenspektrum ist. Die Ähnlichkeit zwischen den Massenspektren kann, wie oben bereits beschrieben, durch unterschiedliche mathematisch-statistische Analyseverfahren ermittelt werden.

Die Erfindung stellt besondere Ausführungsformen des Verfahrens von Govorun mit isotopenmarkierten Nahrungsbausteinen bereit. Im Gegensatz zur Ausführungsform von Demirev et al. wird jedoch ein synthetisches Medium verwendet, in dem nur wenige Aminosäuren, vorzugsweise nur eine einzige Aminosäure, durch den Einbau von ¹³C, ¹⁵N, ¹⁸O oder ³⁴S isotopenmarkiert sind. Sind mehrere Aminosäuren isotopenmarkiert, so sind sie so markiert, dass sie jeweils alle um die gleiche ganzzahlige Masse *Δm* schwerer sind als die entsprechenden unmarkierten Aminosäuren. Statt also, wie bei Demirev et al., zu komplizierten Vorausberechnungen der Verschiebungen von bestimmten "Biomarkern" (oder sogar zu MS/MS-Messungen und Identifizierung dieser Biomarker) gezwungen zu sein, wird in dieser Erfindung ein Medium verwendet, das automatisch zu Peakverschiebungen von *n*×Δ*m* aller Proteine führt, die diese isotopenmarkierten Aminosäuren enthalten, wobei *n* der Anzahl der jeweils isotopenmarkierten Aminosäuren in dem Protein entspricht. Die Massendifferenzen *Δm* der isotopenmarkierten Aminosäuren zu den entsprechenden unmarkierten Aminosäuren sollen zwischen 6 und 12 Dalton, vorzugsweise bei 8 bis 10 Dalton liegen.

Demirev et al. stellen die Resistenz einer Mikrobe dadurch fest, dass verschobene Peaks aufgefunden werden. Es hat sich jedoch in unseren Experimenten herausgestellt, dass nicht-resistente Mikroben im isotopenmarkierten Medium auch in Anwesenheit von Antibiotika eine gewisse Menge an isotopenmarkierten Nahrungsbausteinen aufnehmen können, bevor vollständige Wachstumshemmung oder die strukturelle Zerstörung eintreten. Und resistente Mikroben können auch nach Stunden der Kultivierung noch einen Anteil an unmarkierten Aminosäuren enthalten, der mathematisch unter Kenntnis der Verdoppelungszeit nicht zu erwarten ist.

In beiden erfindungsgemäßen Verfahren kann die Resistenz der Mikroben aus den Quotienten der Intensitäten der verschobenen und nichtverschobenen Peaks (Massensignale) der jeweils gleichen Proteine bestimmt wird. Dieses Vorgehen bedingt aber, dass die verschobenen Peaks den unverschobenen individuell eindeutig zugeordnet und mögliche Überlagerungen mit Peaks anderer Proteine erkannt und berücksichtigt werden können. Es ist dabei günstig, wenn möglichst viele Verschiebungspaare aufgefunden werden können. Neben den Quotienten der Intensitäten kann man auch die relativen oder absoluten Differenzen der Quotienten aus den Peakintensitäten zur Bestimmung der Resistenzen verwenden.

Zwischen 60 bis 85 Prozent der in den Massenspektren gemessenen Proteine gehören zu den ribosomalen Proteinen. In diesen ribosomalen Proteinen sind - wie bei anderen Proteinen auch - die Aminosäuren nicht gleich verteilt, sondern es gibt mit unter fünf Prozent seltener vorkommende und mit über fünf Prozent häufiger vorkommende Aminosäuren (bei Gleichverteilung kämen alle 20 Aminosäuren mit je fünf Prozent vor). Häufig vorkommende Aminosäuren können im oberen gemessenen Massenbereich - im Allgemeinen wird das Massenspektrum von drei bis etwa 15 Kilodalton gemessen - durchaus etwa 20- bis 40-fach in einem einzigen Protein vorkommen, während seltenere Aminosäuren nur bis etwa vier- oder sechsmal auftreten. Die Verschiebungen der Peaks sind somit bei selteneren oder durchschnittlich häufig vorkommenden Aminosäuren sehr viel leichter individuell auffindbar als bei sehr häufig vorkommenden Aminosäuren. Da für die Berechnung der Intensitätsquotienten die einander zugehörigen Peakpaare individuell auffindbar sein müssen, ist es vorzuziehen, eine oder mehrere der seltener oder durchschnittlich häufig vorkommenden Aminosäuren zu markieren.

Die Mikroben einer zu untersuchenden Probe können in beiden erfindungsgemäßen Verfahren in Kulturen mit Zugabe eines Antibiotikums in verschiedenen Konzentrationsabstufungen kultiviert werden, um mit einer quantitativen Auswertung der Massenspektren bei unterschiedlichen Konzentrationen den MHK-Wert des Antibiotikums zu bestimmen oder zumindest abzuschätzen. Im zweiten Verfahren können dabei in Schritt (b) mehrere Kultivierungen mit dem Normalmedium jeweils mit Zugabe verschiedener Antibiotika und/oder mit Zugabe verschiedener Konzentrationen vorgenommen werden.

Das in den erfindungsgemäßen Verfahren verwendete synthetische Medium enthält im Wesentlichen nicht die entsprechende unmarkierte Aminosäuren einer isotopenmarkierten Aminosäure enthält. Im Wesentlichen bedeutet, dass die entsprechende unmarkierte Aminosäure im Verhältnis zur isotopenmarkierten Aminosäure zu weniger als zwanzig Prozent, bevorzugt zu weniger als 5 Prozent, insbesondere zu weniger als ein Prozent oder gar nicht im Medium enthalten ist. Des Weiteren kann das synthetische Medium anstelle eines Antibiotikums auch ein Gemisch verschiedener Antibiotika enthalten. Die Kultivierungsschritte der erfindungsgemäßen Verfahren finden bevorzugt in einem kompakten Flüssigkeitsvolumen (z.B. in einem Zentrifugenröhrchen oder den Töpfchen einer Mikroplatte) statt, können aber auch auf entsprechenden Agarplatten in Petrischalen erfolgen.

Die Erfindung stellt weiterhin ein erfindungsgemäßes Medium zur Kultivierung von Mikroben bereit, das ein synthetisches Medium mit proteinogenen Aminosäuren ist, von denen mindestens eine isotopenmarkiert ist. Es wird dabei die entsprechende unmarkierte Aminosäuren im Wesentlichen vollständig durch die isotopenmarkierte Aminosäure ersetzt, d.h., dass die entsprechende unmarkierte Aminosäure im Verhältnis zur isotopenmarkierten Aminosäure zu weniger als zwanzig Prozent, bevorzugt zu weniger als 5 Prozent, insbesondere zu weniger als ein Prozent oder gar nicht im Medium enthalten ist. Das synthetische Medium ist bevorzugt das Eagle's minimal essential Medium (EMEM) oder Dulbecco's modified Eagle's Medium (DMEM) ist, in dem mindestens eine der Aminosäuren durch eine entsprechende isotopenmarkierte Aminosäure ausgetauscht worden ist. Das synthetische Medium weist weiterhin eine NaCl Konzentration von mehr als 6.4 g/l und/oder Fe(II)SO₄ auf.

### Kurze Beschreibung der Abbildungen

Abbildung 1 zeigt ein Beispiel eines Ablaufdiagramms für die Bestimmung der Resistenz nach einer ersten Ausführungsform der Erfindung.

Abbildung 2 gibt ein Beispiel für ein Ablaufdiagramm nach einer zweiten Ausführungsform wieder.

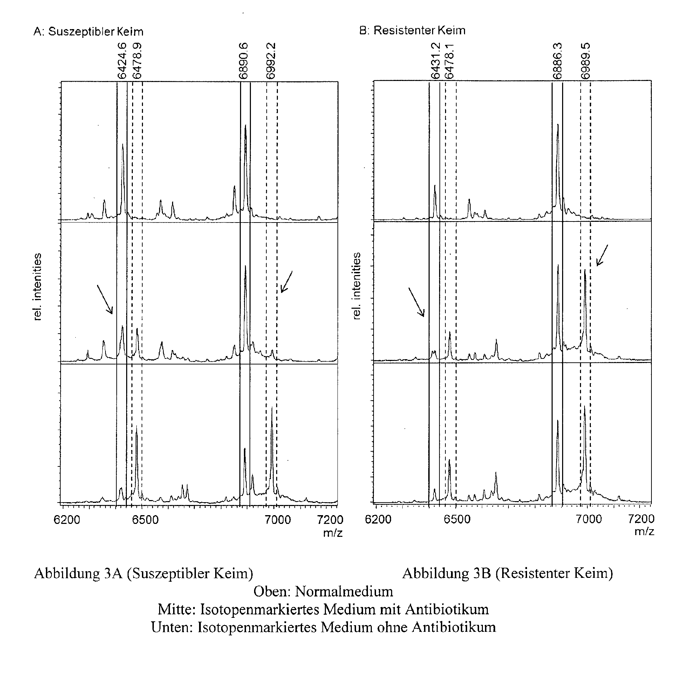

Abbildung 3 zeigt jeweils Ausschnitte aus drei Massenspektren für je ein resistentes und je ein nicht-resistentes (suszeptibles) Bakterium. Oben: Normalmedium; Mitte: Isotopenmarkiertes Medium mit Antibiotikum; Unten: Isotopenmarkiertes Medium ohne Antibiotikum.

### Bevorzugte Ausführungsbeispiele

Alle hier gegebenen Ausführungsformen setzen voraus, dass die Mikroben, deren Resistenz festgestellt werden soll, in genügender Menge hinreichend reiner Form vorliegen. Sie können beispielsweise als Kolonien auf einem Agar oder aber als Mikroben aus einer Blutkultur vorliegen. Bei Agar-Kulturen ist es dabei gängige Praxis, die Mikroben nicht nur einer Kolonie für die Prüfung zu verwenden, sondern die Mikroben aus mindestens fünf Kolonien gemeinsam dieser Prüfung zu unterziehen, um gegebenenfalls auch die Anwesenheit einer resistenten Mikrobe unter nichtresistenten Mikroben der gleichen Art erkennen zu können. Einer geschulten und erfahrenen Fachkraft ist es im Allgemeinen möglich, Kolonien gleicher Mikroben zu erkennen und diese zu sammeln. Sie sind dann zu mischen und für die Kulturen zu teilen. Bei Mikroben aus Blutkulturen, insbesondere aus als positiven Blutkulturen, ist ebenfalls eine Mischung aus den verschiedenartigen Mikroben wahrscheinlich.

Wie oben bereits angemerkt, stellt die Erfindung besonders vorteilhafte Ausführungsformen des Verfahrens von Govorun mit isotopenmarkierten Nahrungsbausteinen bereit. Das Verfahren von Demirev et al. arbeitet mit einem vollständig 13C-markierten und dadurch teuren

Medium und benötigt komplizierte Verfahren zur Vorausbestimmung der Verschiebung einzelner Peaks von Biomarkern. Im Gegensatz dazu wird in dieser Erfindung ein synthetisches Medium verwendet, in dem eine oder wenige Aminosäuren durch ¹³C, ¹⁵N, ¹⁸O und/oder ³⁴S isotopenmarkiert sind, und in dem diese isotopenmarkierten Aminosäuren alle um die gleiche feste Massendifferenz *Δm* schwerer sind als die betreffenden unmarkierten Aminosäuren. In einer Ausführungsform ist sogar nur eine einzige Aminosäure isotopenmarkiert. Dadurch wird erreicht, dass die Massendifferenz *Δm* der Peakpaare in Massenspektren mit und ohne Aufnahme der isotopenmarkierten Nahrungsbausteine immer ein ganzzahliges Vielfaches der Massendifferenz Δ*m* der isotopenmarkierten Aminosäuren zu den unmarkierten Aminosäuren beträgt. (Eine Isotopenmarkierung durch ²D wird hier vermieden, da diese zu leicht durch H/D-Austausch an andere Moleküle abgegeben wird. Eine Isotopenmarkierung durch Phosphor, ebenfalls bei Demirev et al. erwähnt, wird hier weniger bevorzugt verwendet, da es nur ein stabiles Phosphor-Isotop ³¹P gibt.).

Für die Identifizierung der Mikroben werden derzeit überwiegend MALDI-Flugzeitmassenspektrometer im Linearmodus, also ohne Verwendung eines Reflektors, eingesetzt. Um eine hohe Empfindlichkeit zu erhalten, wird die Laserenergie für die Ionisierung sehr hoch gewählt, wodurch aber die meisten der dadurch entstehenden Ionen so stark angeregt werden, dass sie während der Flugzeit zerfallen. Um auch die zerfallenen Teilchen, ob neutral oder noch geladen, ob in der Masse verändert oder nicht, ebenfalls im Massenspektrum messen zu können, werden sie ohne Reflektion am Ende der Flugstrecke gemessen. Ein Sekundärelektronenverstärker kann auch die Neutralteilchen messen. Da aber bei den Zerfällen geringe Mengen an Energie frei werden, die den Zerfallsprodukten als kinetische Energie mitgegeben werden und so ihre Fluggeschwindigkeit in statistischer Weise leicht vermindern oder erhöhen, leidet die Massenauflösung. Die Isotopenverteilungen, die bei guten Flugzeitmassenspektrometern mit Reflektoren bis zu sehr hohen Massen gut in Einzelpeaks aufgelöst werden, bilden hier nur noch einen einzigen, verhältnismäßig breiten Peak.

Wegen dieses geringen Massenauflösungsvermögens muss im unteren Massenbereich des gemessenen Massenspektrums, also bei etwa drei Kilodalton, die Massendifferenz mindestens *Δm* = 6 betragen, um erkennbar zu sein. Günstig sind Massendifferenzen der Aminosäuren mit und ohne Isotopenmarkierung von *Δm* = 6 bis 12 Dalton, vorzugsweise *Δm* = 8 bis 10 Dalton; es können dann auch Peakverschiebungen erkannt werden, die nur von einer isotopenmarkierten Aminosäure stammen. Im oberen Massenbereich, in dem eine noch geringere Massenauflösung vorherrscht, sind in der Regel mehrere isotopenmarkierte Aminosäuren vorhanden, so dass ganz überwiegend nur ein mehrfaches dieser minimalen Massendifferenz *Δm* vorkommt und die Peakverschiebung entsprechend aufgelöst werden kann.

Es sei hier noch angemerkt, dass die überwiegende Zahl aller Peaks im Massenspektrum verschoben wird, wenn eine Resistenz vorliegt. Das Massenspektrum besteht überwiegend aus den Massensignalen von Proteinen (Proteinpeaks). Nur in den seltenen Fällen, in denen ein Protein nicht die Aminosäure enthält, die isotopenmarkiert im Medium vorhanden ist, wird ein Peak nicht verschoben. Auch einige wenige Massensignale, die auf aminosäurefreie Zellbestandteile zurückgehen, werden nicht verschoben. Je größer die Masse, umso wahrscheinlicher muss eine Verschiebung eines Peaks auftreten, da hier überwiegend nur noch Proteine (oder Proteinderivate) als Peaks vorhanden sind, und diese Proteine wegen ihrer Größe mit hoher Wahrscheinlichkeit auch die markierten Aminosäuren enthalten.

Es hat sich experimentell herausgestellt, dass glücklicherweise bei den meisten Mikroben keine Proteine in messbaren Mengen gebildet werden, die eine Mischung aus nichtmarkierten und markierten Aminosäuren der gleichen Art tragen. Das deutet darauf hin, dass die gemessenen Proteine weitgehend neu aus frisch in den aufgenommenen Nahrungsbausteinen enthaltenen isotopenmarkierten Aminosäuren aufgebaut werden, und nur selten aus Aminosäuren, die in dem Mikroben aus Glucose neu synthetisiert werden (Bakterien sind durchaus in der Lage, Aminosäuren aus Kohlenwasserstoffen neu zu bilden. Besonders herangezüchtete Bakterien der Art Corynebacterium glutamicum werden sogar dazu benutzt, jährlich Hunderttausende von Tonnen L-Lysin im Wert von mehr als zwei Milliarden Euro zu produzieren, wobei Melasse als Nahrung dient).

Da nur selten Mischformen vorkommen, treten messbare Verschiebungen eines Peaks fast immer nur um einen festen Betrag von *n*×*Δm* auf, mit fester Anzahl n. Es bilden sich somit ganz überwiegend sauber getrennte Peakpaare.

Ebenso hat sich experimentell herausgestellt, dass bei optimaler Anwendung dieses Verfahrens eine relativ kurze Kultivierungszeit von nur etwa drei Stunden genügt, um die Resistenz zu bestimmen. Das Verfahren ist also außerordentlich schnell; in drei bis vier Stunden nach der Identifizierung einer Mikrobenart kann festgestellt werden, ob die Mikroben resistent sind.

Es gehört zu den besonderen Erkenntnissen, auf denen diese Erfindung beruht, dass besonders bei kurzen Kultivierungszeiten eine individuelle Erkennbarkeit der Peakpaare, die jeweils aus einem nicht verschobenen und einem verschobenen Peak bestehen, sehr wichtig ist. Es wurde nämlich gefunden, dass nicht-resistente Mikroben im isotopenmarkierten Medium auch in Anwesenheit von Antibiotika eine gewisse Menge an isotopenmarkierten Nahrungsbausteinen aufnehmen können, bevor vollständige Wachstumshemmung oder die strukturelle Zerstörung eintreten. Darüber hinaus können in einer noch nicht aufgeklärten Weise resistente Mikroben auch nach Stunden der Kultivierung noch größere Mengen an Proteinen enthalten, die aus durchweg unmarkierten Aminosäuren aufgebaut sind, als mathematisch unter Kenntnis der Verdoppelungszeit zu erwarten sind. Es ist daher besonders bevorzugt, die Resistenz aus den Quotienten der Intensitäten der isotopenverschobenen Peaks zu den jeweils unverschobenen Peaks zu bestimmen. Es ist besonders günstig, viele Quotienten aus den Intensitäten der isotopenverschobenen Peaks zu den jeweils unverschobenen Peaks zu berechnen und daraus die Resistenz mit erhöhter Sicherheit abzuleiten. Dieses Vorgehen bedingt aber, dass es möglich sein muss, die verschobenen Peaks den unverschobenen individuell eindeutig zuzuordnen. Außerdem ist es wichtig, mögliche Überlagerungen der verschobenen Peaks mit Peaks anderer Proteine, die im nicht-isotopenmarkierten Spektrum erscheinen, zu erkennen und diese überlagerten Peaks gegebenenfalls von der Berechnung auszunehmen oder die Überlagerung bei den Berechnungen zu berücksichtigen.

Für die individuelle Erkennbarkeit der Peakpaare ist es wichtig, dass sie nicht zu weit auseinander liegen. Es ist einsehbar leichter, den Partner eines Peaks nur an etwa fünf Stellen suchen zu müssen, als an 20 bis 40 Stellen, die entsprechend um einige Hundert Dalton weit im Massenspektrum entfernt sind und möglicherweise zahlreiche andere verschobene und nichtverschobene Peaks dazwischen enthalten. Um keine großen Massendifferenzen zu erhalten, dürfen weder viele Aminosäuren isotopenmarkiert sein, noch dürfen die markierten Aminosäuren in großer Zahl in einem Protein vorkommen.

Es ist bekannt, dass zwischen 60 bis 85 Prozent der in den Massenspektren gemessenen Proteine zu den ribosomalen Proteinen gehören. Bei einer Gleichverteilung der Aminosäuren kämen alle 20 Aminosäuren in einem Protein mit je fünf Prozent vor. In diesen ribosomalen Proteinen sind aber - sogar stärker als bei anderen Proteinen - die Aminosäuren nicht gleich verteilt, sondern es gibt mit unter fünf Prozent seltener vorkommende und mit über fünf Prozent häufiger vorkommende Aminosäuren. Sehr häufig vorkommende Aminosäuren (wie zum Beispiel Lysin) können in ribosomalen Proteinen durchaus 15 bis 30 Prozent aller Aminosäuren ausmachen. Diese können im oberen gemessenen Massenbereich - im Allgemeinen wird das Massenspektrum auf den Bereich von drei bis etwa 15 Kilodalton beschränkt - durchaus etwa 20- bis 40-fach in einem Protein vorkommen, während seltenere oder durchschnittlich häufige Aminosäuren nur etwa 3- bis 6-mal auftreten. Im Bereich von 15 Kilodalton besitzen die Proteine rund 120 bis 140 Aminosäuren, im Massenbereich um 3000 Dalton nur 25 bis 30 Aminosäuren. Eine mit fünf Prozent durchschnittlich häufige Aminosäure kommt also im unteren Massenbereich im Durchschnitt nur etwa einmal, im oberen Massenbereich sechs- bis siebenmal vor. Die Verschiebungen der Peaks sind somit bei einer selteneren oder durchschnittlich häufigen Aminosäure viel kleiner und sehr viel leichter individuell auffindbar als bei häufig vorkommenden Aminosäuren. Da für die Berechnung der Intensitätsquotienten, die sich als die bevorzugte Form der Auswertung erwiesen hat, die einander zugehörigen Peakpaare individuell auffindbar sein müssen, ist es vorzuziehen, eine seltener oder durchschnittlich häufig vorkommende Aminosäure, beispielsweise Leucin, zu markieren.

Es kann darüber hinaus günstig sein, mehrere Aminosäuren isotopenmarkiert zu verwenden, beispielsweise zwei oder drei, maximal vier. Diese können beispielsweise so ausgesucht werden, dass sie in summa weniger als fünf bis zehn Prozent aller Aminosäuren der ribosomalen Proteine ausmachen, dass aber nach Möglichkeit jedes ribosomale Protein mindestens eine dieser Aminosäuren enthält. Für eine gute Erkennbarkeit der Verschiebung ist es wieder notwendig, dass alle isotopenmarkierten Aminosäuren die gleiche Massendifferenz *Δm* zu den entsprechenden unmarkierten Aminosäuren aufweisen. Es wird dadurch erreicht, dass praktisch alle Peaks, jedenfalls soweit sie überhaupt von Proteinen gebildet werden, bei Vorliegen einer Resistenz verschoben werden.

Wie oben schon angemerkt, ist es eine bevorzugte Art der Bestimmung der Resistenzen, die Intensitätsquotienten für die einzelnen Peakpaare der Verschiebung zu berechnen. Die Intensitätsquotienten können einzeln mit aus Erfahrung gewonnen Schwellenwerten verglichen oder zunächst gemittelt werden, um den Mittelwert mit einem Erfahrungs-Schwellenwert zu vergleichen. Ist der Quotient aus der Intensität des verschobenen zu der des unverschobenen Peaks gebildet, so geben Quotienten oberhalb des Schwellenwerts eine Resistenz an. Der Vergleich der Einzelquotienten hat den Vorteil, dass herausfallende Werte erkannt werden können, und dass sogar massenabhängige Schwellenwerte verwendet werden können.

Es sei noch angemerkt, dass man zur Bestimmung der Resistenzen neben den Quotienten aus den Intensitäten der verschobenen und nicht verschobenen Peaks man auch die relativen oder absoluten Differenzen der Quotienten aus den Peakintensitäten verwenden kann.

Es wurden auch Berechnungsverfahren für die Bestimmung von Resistenzen erprobt, die nicht auf der individuellen Erkennung der Peakpaare beruhen. So kann beispielsweise mit bekannten Algorithmen die Verschiebung des Schwerpunkts des Massenspektrums berechnet werden. Diese Verschiebung des Schwerpunkts sollte ein Maß für die Resistenz sein, besonders, wenn im Gegensatz zu den oben gegebenen Empfehlungen sehr häufige Aminosäuren, oder sogar mehrere häufige Aminosäuren isotopenmarkiert sind. Es hat sich jedoch ergeben, dass dieses Berechnungsverfahren nicht immer mit gewünschter Sicherheit die Resistenz anzeigt. Da immer nur ein Ausschnitt des Massenspektrums (beispielsweise von drei bis 15 Kilodalton) gemessen wird, ist durchaus zu erwarten, dass an der Untergrenze verschobene Peak in den Massenbereich hineinwandern, und an der Obergrenze herauswandern, wodurch die Schwerpunktsberechnung verfälscht wird.

Um die MHK-Werte (minimale Hemmkonzentrationen) der Antibiotika bestimmen oder abschätzen zu können, können Kulturen mit Zugabe eines Antibiotikums in verschiedenen Konzentrationsabstufungen verwendet werden. Die für bestimmte Mikroben gängigen MHK-Werte der verschiedenen Antibiotika sind durchaus bekannt; es gibt aber gelegentlich starke und überraschende Abweichungen. Um diese MHK-Werte grob zu überprüfen, können verschiedenen Medien Antibiotika in verschiedenen Konzentrationsstufen beigegeben werden, die beispielsweise den Konzentration 1*MHK, 10*MHK und 100*MHK der bisher bekannten MHK-Werte entsprechen können. Erfahrungsgemäß lassen sich mit dem oben geschilderten Verfahren die Wirkung der Konzentration 1*MHK gerade noch nicht, die Wirkung von 10*MHK deutlich, und die Wirkung von 100*MHK als sehr stark feststellen. Die Wirkung kann beispielsweise an den Werten der einzelnen oder gemittelten Intensitätsquotienten abgelesen werden. Wird das Verfahren ohne Konzentrationsabstufungen durchgeführt, so hat sich für die erfindungsgemäßen Verfahren eine Konzentration des Antibiotikums von 10*MHK als besonders günstig erwiesen.

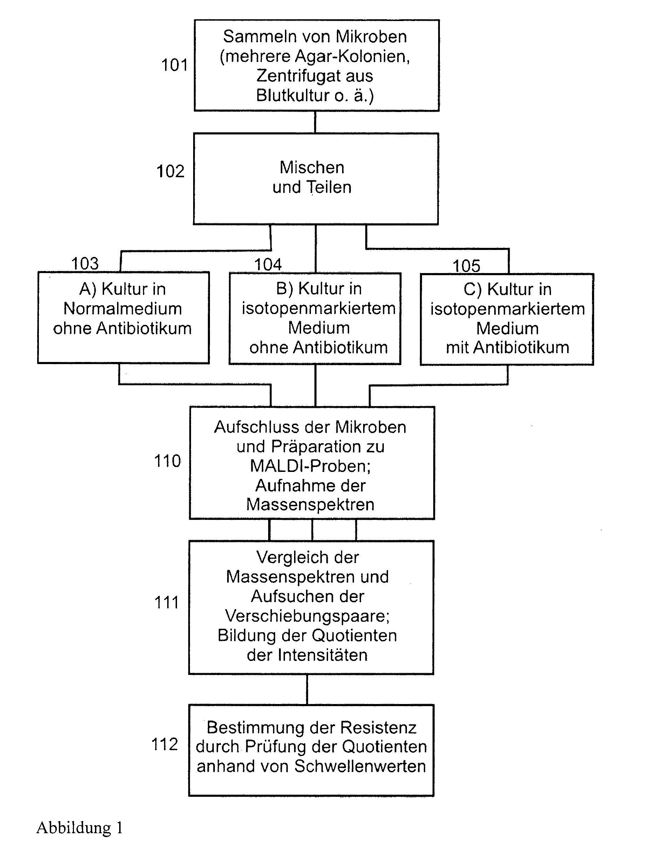

Der Ablauf einer ersten bevorzugten Ausführungsform zur Bestimmung von Resistenzen ist im Diagramm der Abbildung 1 wiedergegeben. Zunächst werden die Mikroben gesammelt (101), gemischt und für die verschiedenartigen Kulturen geteilt (102). Sie werden dann in einem Medium ohne Isotopenmarkierung und ohne Antibiotika (103), einem Medium mit Isotopenmarkierung, aber ohne Antibiotika (104) und einem Medium mit Isotopenmarkierung und mit Antibiotikum (105) kultiviert. Die Mikroben aus den drei Kulturen werden dann zu MALDI-Proben verarbeitet, und es werden Massenspektren aufgenommen (110). Es werden die Verschiebungspaare gesucht und die Quotienten der Intensitäten der jeweiligen Peakpartner gebildet (111). Durch den Vergleich der Quotienten mit Schwellenwerten, die als Erfahrungswerte vorliegen, wird die Resistenz bestimmt (112).

Für die Prüfung der Resistenz gegenüber mehreren Antibiotika müssen nach dieser Ausführungsform der Erfindung mehrere Kulturen mit mehreren Antibiotika angesetzt werden, erforderlichenfalls sogar mit jeweils verschiedenen Konzentrationsabstufungen der Antibiotika. Der Verbrauch eines isotopenmarkierten Mediums (jeweils mit mindestens einer isotopenmarkierten Aminosäure) ist bei einer solch großen Zahl von Kulturen teuer. Daher wird hier eine weitere, abgewandelte Ausführungsform vorgeschlagen, die zwar etwas längere Zeit braucht, aber weit kostengünstiger ist, besonders dann, wenn die Resistenz gegenüber mehreren Antibiotika geprüft werden soll. Es kann im Einzelfall entschieden werden, ob der Zeitgewinn der obig beschriebenen Ausführungsform oder der Kostenvorteil der nun geschilderten Ausführungsform in Anspruch genommen werden soll. Es soll hier noch angemerkt werden, dass Zeit und Kosten der massenspektrometrischen Messung und Bestimmung gegenüber dem Zeitverbrauch der Kultivierungen der verschiedenen Ausführungsformen und den Kosten der Medien praktisch kaum ins Gewicht fällt. Die massenspektrometrische Messung dauert nur Minuten.

Eine zweite bevorzugte Ausführungsform besteht darin, zunächst in einem ersten Schritt a) in einer ersten Kultur in einem Medium mit einer kleinen Anzahl von maximal vier, vorzugsweise jedoch weniger, isotopenmarkierten Aminosäuren genügend Mikroben zu generieren, bei denen die betreffenden Aminosäuren praktisch vollständig als isotopenmarkierte Aminosäuren vorhanden sind. Die isotopenmarkierten Aminosäuren sollen zu den selteneren oder durchschnittlich häufigen Aminosäuren gehören und einen Gesamtanteil von nur etwa fünf bis maximal zehn Prozent aller Aminosäuren in ribosomalen Proteinen ausmachen. Auch hier soll der Massenunterschied *Δm* zwischen isotopenmarkierten und unmarkierten Aminosäuren für alle verwendeten Aminosäuren gleich sein. Für diese Ausführungsform ist es günstig, mit einer möglichst hohen Ausgangsmenge an Mikroben zu arbeiten (d.h., möglichst viele Kolonien), um mit einer Kultivierungsdauer von etwa vier Stunden auszukommen. Es kann hier wieder ein Gemisch aus proteinogenen Aminosäuren, Kohlenwasserstoffen wie beispielsweise Glucose, und den notwendigen Vitaminen und Mineralien eingesetzt werden, wobei das Gemisch auf ein möglichst günstiges Wachstum der Mikroben einzustellen ist.

Im zweiten Schritt b) dieser Ausführungsform werden die isotopenmarkierten Mikroben dann in preiswerten Normalmedien kultiviert, die gleichzeitig auch optimale Wachstumsbedingungen bieten. Die isotopenmarkierten Mikroben können nun zeitgleich in einem Normalmedium ohne Antibiotika und in mehreren Normalmedien mit Antibiotika, erforderlichenfalls mit jeweils mehreren Konzentrationsabstufungen kultiviert werden. Werden ein Normalmedium ohne Antibiotika und Normalmedien mit vier Antibiotika in je drei Konzentrationsstufen verwendet, so sind zeitgleich 13 Kulturen anzusetzen. Resistente Mikroben bilden bei Teilungen und Wachstum neue Proteine mit unmarkierten Aminosäuren, wodurch die isotopenmarkierten Aminosäuren in wenigen Generationen immer weiter verdünnt werden; nicht-resistente Mikroben ohne wesentliches Wachstum behalten den Großteil ihrer Proteine mit isotopenmarkierten Aminosäuren. Auch hier ist es günstig, durch die Isotopenmarkierung nur einer oder wenigen selteneren oder durchschnittlich häufigen Aminosäuren die Peakpaare leicht individuell identifizieren und die entsprechende Quotienten berechnen zu können. Die Kultivierung ergibt nach etwa drei Stunden in einem dritten Schritt c) Massenspektren, die die Resistenzen sicher anzeigen. Je nach dem zellulären Wirkungsort des Antibiotikums kann diese Zeit auch kürzer sein, z. B. bei Aminoglycosiden, die direkt in die Proteinbiosynthese eingreifen.

Die Art der Auswertung zur Bestimmung der Resistenzen kann analog zu der Auswertung gestaltet werden, die in der ersten Ausführungsform angewendet wurde; es ist jedoch zu beachten, dass die Massenverschiebung der Peaks jetzt zu kleineren Massen hin erfolgt.

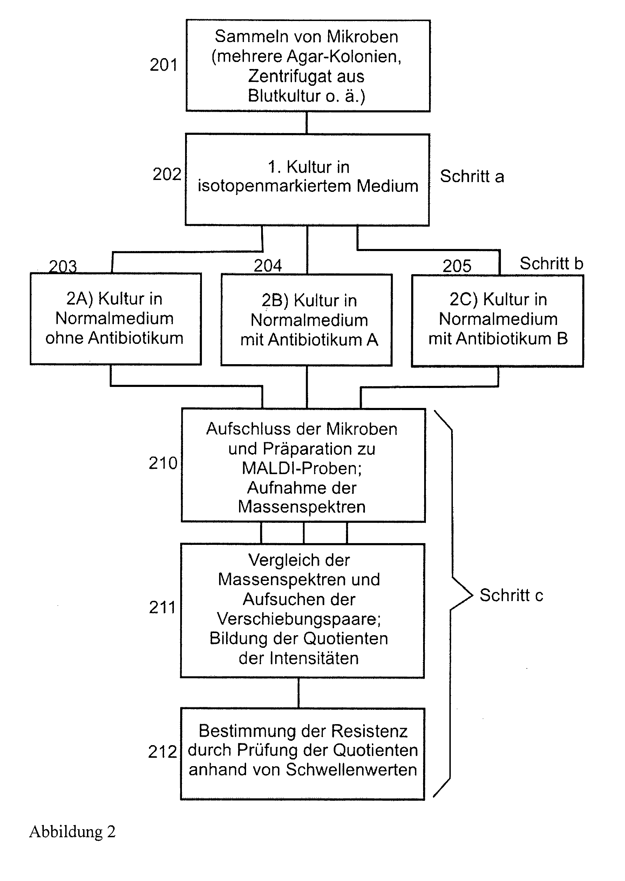

Der Ablauf dieser zweiten Ausführungsform ist im Diagramm der Abbildung 2 wiedergegeben. Wieder werden zunächst Mikroben gesammelt (201). Diese Mikroben werden dann in einem ersten Schritt a) in einem ersten Medium mit einigen isotopenmarkierten Aminosäuren soweit gezüchtet (202), dass sie praktisch durchgängig nur noch diese Aminosäuren in isotopenmarkierter Form besitzen, nicht mehr in deren unmarkierter Form. Im Schritt b) werden nun die isotopenmarkierten Mikroben in mehreren Normalmedien weitergezüchtet: in einem Medium ohne Antibiotikum (203), in weiteren Medien mit Antibiotikum A (204), Antibiotikum B (205) usw.. Im Schritt c) werden Massenspektren der Mikroben aus den verschiedenen Kulturen aufgenommen (210), die Quotienten aus den Intensitäten der Peakpaare gebildet (211) und aus dem Vergleich der Quotienten mit Schwellenwerten die Resistenz bestimmt (212).

In beiden Ausführungsformen kann die Kultivierung jeweils in Zentrifugenröhrchen (beispielsweise Eppendorf-Röhrchen) oder in Filterplatten (beispielsweise Acropep 96-well Filterplatten) vorgenommen werden. Die weiteren Aufbereitungen der Mikroben für eine Messung als MALDI-Proben sind dem Fachmann bekannt (MALDI = Ionisierung durch matrixunterstützte Laserdesorption).

Es wurde bisher alles auf eine Ionisierung durch MALDI in einem MALDI-Flugzeitmassenspektrometer abgestimmt. MALDI hat den großen Vorteil, ganz überwiegend einfach geladene Molekül-Ionen zu bilden. Damit wird die Struktur der Massenspektren einfach und das Auffinden von Peakverschiebungen leicht. Das heißt aber nicht, dass nicht auch andere Arten der Ionisierung eingesetzt werden könnten. Die sprüh-basierten Verfahren wie ESI (Elektrosprüh-Ionisierung) oder DESI (direkte Oberflächen-Ionisierung fester Proben durch Elektrosprühen) haben aber den Nachteil, zu viele vielfach geladene Ionen zu bilden, die die Massenspektren überladen. Es gibt jedoch auch andere Ionisierungsverfahren, die überwiegend einfach geladene Ionen erzeugen, wie beispielsweise die chemische Ionisierung. Die chemische Ionisierung kann in Verbindung mit Neutral-Sprühverfahren, aber auch mit Laser-Ablation von festen Proben eingesetzt und in Verbindung mit einem OTOF (Flugzeitmassenspektrometer mit orthogonalem Ioneneinschuss) verwendet werden. Die so erhaltenen Massenspektren bieten bei hoher Empfindlichkeit höchste Massenauflösung (siehe beispielsweise J. Franzen und K. Michelmann, DE 10 2005 044 307 B4). Selbstredend können auch andere Arten von Massenspektrometern eingesetzt werden, wenn sie den bevorzugten Massenbereich für die Spektrenmessung bieten. Auch eine Vortrennung der Mikrobenproteine durch Separationsverfahren wie HPLC oder Elektrophorese ist möglich, erscheint aber wegen der stark verlängerten Dauer der Analysen nicht sehr günstig.

Als Kultivierungsmedium kann beispielsweise von DMEM (Dulbecco's modified Eagle medium) ausgegangen werden, das es auch in Formen gibt, in denen einzelne (auch mehrere) Aminosäuren fehlen, so dass isotopenmarkierte Aminosäuren zugesetzt werden können. DMEM ist für die Aufzucht von eukariotischen Zellkulturen entwickelt worden und enthält neben den gewünschten Aminosäuren bereits Mineralien (Ca, Fe, K, Mg, Na in verschiedenen Formen), Kohlenhydrate (L-Glucose, Natriumpyruvat) und sieben verschiedene Vitamine, die für eukariotische Zellen wichtig sind. Für die Kultivierung von Mikroben ist hat es sich jedoch in Experimenten als zweckmäßig erwiesen, weitere Mineralien (für manche Bakterien NaCl in höheren Mengen, insbesondere aber Fe(II)SO₄), Glucose und Vitamine zuzusetzen, um ein besseres Wachstum der Mikroben zu erzielen. Schließlich sind die isotopenmarkierten Aminosäuren hinzuzufügen, die aber bevorzugt nicht schon in unmarkierter Form vorhanden sein dürfen. Die für das Wachstum von Mikroben optimierten Medien können insbesondere in gefriergetrockneter Form in geeigneten Packungsgrößen hergestellt und kommerziell für die Verwendung zur massenspektrometrischen Bestimmung der Resistenzen angeboten werden.

Auch die Medien mit Zugaben von Antibiotika können in ähnlicher Weise vorbereitet kommerziell angeboten werden.

## Patentansprüche

1. Verfahren zur massenspektrometrischen Bestimmung der Resistenz von Mikroben gegen ein Antibiotikum, in dem die Mikroben aus einer zu untersuchenden Probe in einer ersten Kultur mit Zugabe eines Antibiotikums und einer zweiten Kultur ohne Zugabe eines Antibiotikums kultiviert werden, wobei in beiden Kulturen ein Medium mit isotopenmarkierten Nahrungsbausteine verwendet wird, und danach Massenspektren der Mikroben aus den beiden Kulturen aufgenommen und miteinander verglichen werden, **dadurch gekennzeichnet, dass** das Medium ein synthetisches Medium ist, das proteinogene Aminosäuren enthält, dass mindestens eine der Aminosäuren isotopenmarkiert ist und alle isotopenmarkierten Aminosäuren die gleiche ganzzahlige Massendifferenz *Δm* zu den entsprechenden unmarkierten Aminosäuren aufweisen und dass die entsprechende unmarkierte Aminosäure im Verhältnis zur isotopenmarkierten Aminosäure zu weniger als zwanzig Prozent im synthetischen Medium enthalten ist.

2. Verfahren nach Anspruch 1, **dadurch gekennzeichnet, dass** nur eine Aminosäure isotopenmarkiert ist und diese Aminosäure in ribosomalen Proteinen der Mikroben weniger als fünf Prozent ausmacht.

3. Verfahren nach Anspruch 1, **dadurch gekennzeichnet, dass** mehrere Aminosäuren isotopenmarkiert sind und dass diese isotopenmarkierten Aminosäuren in ribosomalen Proteinen der Mikroben insgesamt weniger als zehn Prozent ausmachen.

4. Verfahren nach Anspruch einem der Ansprüche 1 bis 3, **dadurch gekennzeichnet, dass** die Mikroben in einer dritten Kultur in dem synthetischen Medium mit unmarkierten Aminosäuren und ohne Zugabe des Antibiotikums kultiviert werden, ein Massenspektrum der Mikroben aus der dritten Kultur aufgenommen wird und das Massenspektrum der ersten Kultur mit dem Massenspektrum der dritten Kultur verglichen wird, wobei die Mikroben als resistent ausgewiesen werden, wenn das Massenspektrum der ersten Kultur dem Massenspektrum der zweiten Kultur ähnlicher als dem Massenspektrum der dritten Kultur ist.

5. Verfahren zur massenspektrometrischen Bestimmung der Resistenz von Mikroben gegen ein Antibiotikum, **mit den Schritten:**
(a) Kultivierung der Mikroben in einem synthetischen Medium, in dem mindestens eine der Aminosäuren isotopenmarkiert ist und alle isotopenmarkierten Aminosäuren in dem Medium die gleiche ganzzahlige Massendifferenz *Δm* zu den entsprechenden unmarkierten Aminosäuren aufweisen und die entsprechende unmarkierte Aminosäure im Verhältnis zur isotopenmarkierten Aminosäure zu weniger als zwanzig Prozent enthalten ist,
(b) Kultivierung der in Schritt a) kultivierten Mikroben in einer ersten Kultur mit Zugabe eines Antibiotikum und einer zweiten Kultur ohne Zugabe eines Antibiotikums, in beiden Kulturen jeweils mit einem Normalmedium ohne isotopenmarkierte Nahrungsbausteine, und
(c) Bestimmung der Resistenz gegenüber dem Antibiotikum durch Aufnahme und Vergleich von Massenspektren der Mikroben der ersten und zweiten Kultur.

6. Verfahren nach Anspruch 5, **dadurch gekennzeichnet, dass** in Schritt a) nur eine Aminosäure isotopenmarkiert ist und diese Aminosäure in ribosomalen Proteinen der Mikroben weniger als fünf Prozent ausmacht.

7. Verfahren nach Anspruch 5, **dadurch gekennzeichnet, dass** in Schritt a) mehrere Aminosäuren isotopenmarkiert sind und dass diese isotopenmarkierten Aminosäuren in ribosomalen Proteinen der Mikroben insgesamt weniger als zehn Prozent ausmachen.

8. Verfahren nach einem der Ansprüche 5 bis 7, **dadurch gekennzeichnet, dass** ein drittes Massenspektrum von den in Schritt a) kultivierten Mikroben aufgenommen wird und mit dem Massenspektrum der ersten Kultur verglichen wird, wobei die Mikroben als resistent ausgewiesen werden, wenn das Massenspektrum der ersten Kultur dem Massenspektrum der zweiten Kultur ähnlicher als dem dritten Massenspektrum ist.

9. Verfahren nach einem der Ansprüche 5 bis 8, **dadurch gekennzeichnet, dass** in Schritt b) mehrere Kultivierungen mit dem Normalmedium jeweils mit Zugabe verschiedener Antibiotika und/oder mit Zugabe verschiedener Konzentrationen vorgenommen werden.

10. Verfahren nach einem der Ansprüche 1 bis 9, **dadurch gekennzeichnet, dass** die Resistenz der Mikroben aus den Quotienten der Intensitäten der verschobenen und nichtverschobenen Peaks (Massensignal) der jeweils gleichen Proteine bestimmt wird.

11. Medium zur Kultivierung von Mikroben, **dadurch gekennzeichnet, dass** das Medium ein synthetisches Medium mit proteinogenen Aminosäuren ist, von denen mindestens eine isotopenmarkiert ist, wobei das synthetische Medium Fe(II)SO₄ aufweist und die entsprechende unmarkierte Aminosäure im Verhältnis zur isotopenmarkierten Aminosäure zu weniger als zwanzig Prozent enthält.

12. Medium nach Anspruch 11, **dadurch gekennzeichnet, dass** das synthetische Medium das Eagle's minimal essential Medium (EMEM) oder Dulbecco's modified Eagle's Medium (DMEM) ist, in dem mindestens eine der Aminosäuren durch eine entsprechende isotopenmarkierte Aminosäure ausgetauscht worden ist und das eine NaCl Konzentration von mehr als 6.4 g/l und Fe(II)SO₄ aufweist.

## Claims

1. Method for the mass spectrometric determination of the resistance of microbes against an antibiotic, where the microbes from a sample under investigation are cultivated in a first culture to which an antibiotic is added and in a second culture to which no antibiotic is added, while a medium with isotope-labeled nutrient components is used in both cultures, and afterwards mass spectra of the microbes from both cultures are acquired and compared with each other, **wherein** the medium is a synthetic medium which contains proteinogenic amino acids, and at least one of the amino acids is isotope-labeled, and all the isotope-labeled amino acids have the same integer mass difference *Δm* in relation to the corresponding unlabeled amino acids, and the ratio of the corresponding unlabeled amino acid to the isotope-labeled amino acid contained in the synthetic medium is less than twenty percent.

2. Method according to Claim 1, wherein only one amino acid is isotope-labeled and this amino acid amounts to less than five percent in ribosomal proteins of the microbes.

3. Method according to Claim 1, wherein several amino acids are isotope-labeled and these isotope-labeled amino acids amount to less than ten percent overall in ribosomal proteins of the microbes.

4. Method according to one of the Claims 1 to 3, wherein the microbes are cultivated in a third culture in the synthetic medium with unlabeled amino acids and without the addition of the antibiotic, a mass spectrum of the microbes from the third culture is acquired, and the mass spectrum of the first culture is compared to the mass spectrum of the third culture. The microbes are identified as being resistant if the mass spectrum of the first culture has a greater similarity to the mass spectrum of the second culture than to the mass spectrum of the third culture.

5. Method for the mass spectrometric determination of microbial resistance against an antibiotic, **comprising the steps:**
(a) Cultivation of the microbes in a synthetic medium in which at least one of the amino acids is isotope-labeled, and where all the isotope-labeled amino acids in the medium have the same integer mass difference *Δm* in relation to the corresponding unlabeled amino acids, and the ratio of the corresponding unlabeled amino acid to the isotope-labeled amino acid contained in the medium is less than twenty percent,
(b) Cultivation of the microbes cultivated in Step a) in a first culture with the addition of an antibiotic and a second culture without the addition of an antibiotic, in both cultures with a normal medium without isotope-labeled nutrient components, and
(c) Determination of the resistance against the antibiotic by acquiring and comparing mass spectra of the microbes from the first and the second culture.

6. Method according to Claim 5, wherein in Step a) only one amino acid is isotope-labeled and this amino acids amounts to less than five percent in ribosomal proteins of the microbes.

7. Method according to Claim 5, wherein in Step a) several amino acids are isotope-labeled and these isotope-labeled amino acids amount to less than ten percent overall in ribosomal proteins of the microbes.

8. Method according to one of the Claims 5 to 7, wherein a third mass spectrum is acquired from the microbes cultivated in Step a) and compared to the mass spectrum of the first culture, the microbes being identified as resistant if the mass spectrum of the first culture has a greater similarity to the mass spectrum of the second culture than to the third mass spectrum.

9. Method according to one of the Claims 5 to 8, wherein at Step b) several cultivations are carried out with the normal medium, in each case with the addition of different antibiotics and/or the addition of different concentrations.

10. Method according to one of the Claims 1 to 9, wherein the resistance of the microbes is determined from the ratios of the intensities of the shifted and unshifted peaks (mass signals) of the same proteins in each case.

11. Medium for the cultivation of microbes, wherein the medium is a synthetic medium with proteinogenic amino acids of which at least one is isotope-labeled, where the synthetic medium contains Fe(II)SO₄ and the ratio of the corresponding unlabeled amino acid to the isotope-labeled amino acid contained therein is less than twenty percent.

12. Medium according to Claim 11, wherein the synthetic medium is Eagle's minimal essential Medium (EMEM) or Dulbecco's modified Eagle's Medium (DMEM), in which at least one of the amino acids has been replaced by a corresponding isotope-labeled amino acid and which has a NaCl concentration of more than 6.4 g/l and contains Fe(II)SO₄.

## Revendications

1. Méthode de la spectrométrie de masse utilisée pour déterminer la résistance de microbes à un antibiotique, consistant à cultiver les microbes issus d'un échantillon à analyser dans une première culture à laquelle est ajoutée un antibiotique et dans une deuxième culture sans l'ajout d'un antibiotique et à utiliser pour les deux cultures un milieu contenant des composants alimentaires marqués d'un isotope, puis à enregistrer et comparer entre eux les spectres de masse des microbes issus des deux cultures, **caractérisée en ce que** le milieu est un milieu synthétique contenant des acides aminés protéinogènes, qu'au moins l'un des acides aminés est marqué d'un isotope et que tous les acides aminés marqués d'un isotope présentent la même différence de masse entière *Δm* par rapport aux acides aminés non marqués d'un isotope et que l'acide aminé correspondant non marqué est présent pour moins de 20 % dans le milieu synthétique par rapport à l'acide aminé marqué d'un isotope.

2. Méthode selon la revendication 1, **caractérisée en ce que** seulement un acide aminé est marqué d'un isotope et que cet acide aminé représente moins de 5 % dans les protéines ribosomiques des microbes.

3. Méthode selon la revendication 1, **caractérisée en ce que** plusieurs acides aminés sont marqués d'un isotope et que ces acides aminés marqués d'un isotope représentent au total moins de 10 % dans les protéines ribosomiques des microbes.

4. Méthode selon l'une des revendications 1 à 3, **caractérisée en ce que** les microbes sont cultivés dans une troisième culture dans le milieu synthétique avec des acides aminés non marqués et sans l'ajout de l'antibiotique, qu'un spectre de masse des microbes est enregistré dans la troisième culture et que le spectre de masse de la première culture est comparé avec le spectre de masse de la troisième culture, les microbes étant dits résistants si le spectre de masse de la première culture ressemble plus au spectre de masse de la deuxième culture qu'au spectre de masse de la troisième culture.

5. Méthode de la spectrométrie de masse utilisée pour déterminer la résistance de microbes à un antibiotique, **avec les étapes suivantes :**
(a) Culture des microbes dans un milieu synthétique dans lequel au moins l'un des acides aminés est marqué d'un isotope et tous les acides aminés marqués d'un isotope dans le milieu présentent la même différence de masse entière *Δm* par rapport aux acides aminés non marqués correspondants, et dans lequel l'acide aminé correspondant non marqué est présent pour moins de 20 % par rapport à l'acide aminé marqué d'un isotope ;
(b) Culture des microbes cultivés dans l'étape a) dans une première culture à laquelle est ajouté un antibiotique et dans une deuxième culture sans l'ajout d'un antibiotique, avec dans chacune des deux cultures un milieu normal sans composants alimentaires marqués d'un isotope ; et
(c) Détermination de la résistance à l'antibiotique par l'enregistrement et la comparaison de spectres de masse des microbes de la première et de la deuxième culture.

6. Méthode selon la revendication 5, **caractérisée en ce que** dans l'étape a) seulement un acide aminé est marqué d'un isotope et que cet acide aminé représente moins de 5 % dans les protéines ribosomiques des microbes.

7. Méthode selon la revendication 5, **caractérisée en ce que** dans l'étape a) plusieurs acides aminés sont marqués d'un isotope et que ces acides aminés marqués d'un isotope représentent au total moins de 10 % dans les protéines risobomiques des microbes.

8. Méthode selon l'une des revendications 5 à 7, **caractérisée en ce qu'**un troisième spectre de masse est enregistré à partir des microbes cultivés dans l'étape a) et comparé avec le spectre de masse de la première culture, les microbes étant dits résistants si le spectre de masse de la première culture ressemble plus au spectre de masse de la deuxième culture qu'au troisième spectre de masse.

9. Méthode selon l'une des revendications 5 à 8, **caractérisée en ce que** dans l'étape b) plusieurs cultures sont effectuées avec le milieu normal, avec pour chaque culture l'ajout de différents antibiotiques et/ou l'ajout de différentes concentrations.

10. Méthode selon l'une des revendications 1 à 9, **caractérisée en ce que** la résistance des microbes est déterminée à partir du rapport des intensités des pics déplacés et non déplacés (signal de masse) des protéines de même type.

11. Milieu utilisé pour cultiver des microbes, **caractérisé en ce que** le milieu est un milieu synthétique avec des acides aminés protéinogènes dont un au moins est marqué d'un isotope, le milieu synthétique présentant du Fe(II)SO₄ et l'acide aminé correspondant non marqué étant présent dans le milieu synthétique pour moins de 20 % par rapport à l'acide aminé marqué d'un isotope.

12. Milieu selon la revendication 11, **caractérisé en ce que** le milieu synthétique est le milieu essentiel minimum de Eagle (EMEM) ou le milieu de Eagle modifié par Dulbecco (DMEM) dans lequel au moins l'un des acides aminés a été échangé contre un acide aminé correspondant marqué d'un isotope et qui présente une concentration en NaCl de plus de 6,4 g/l et du Fe(II)SO₄.
